# EUROPEAN PATENT APPLICATION

(11) **EP 2 392 338 A1**
(43) Date of publication of application: **07.12.2011**
(21) Application number: 10735971.3
(22) Date of filing: 29.01.2010
(51) Int. Cl.: A61K 31/593, A61K 31/426, A61P 1/02, A61P 3/14, A61P 19/00, A61P 19/02, A61P 19/08, A61P 19/10, A61P 29/00, A61P 35/00, A61P 35/04

(54) **PROPHYLACTIC OR THERAPEUTIC AGENT FOR DISEASES ASSOCIATED WITH ABNORMAL BONE METABOLISM**

(30) Priority: 30.01.2009 JP 2009019644
(71) Applicant: Teijin Pharma Limited, Tokyo 100-0013 (JP); Dong Wha Pharmaceutical Co., Ltd., Seoul 110-130 (KR)
(72) Inventor: OCHIAI, Eiji, Hino-shi Tokyo 191-0065 (JP); NAKAYAMA, Yasunori, Hino-shi Tokyo 191-0065 (JP); LEE, Jin-Soo, Yongin-Si Kyunggi-Do 449-531 (KR); HWANG, Yun-Ha, Gunpo-Si Kyunggi-Do 435-730 (KR); JEONG, Doc-Gyun, Seoul 157-011 (KR)
(74) Representative: Hallybone, Huw George
(86) International application number: PCT/JP2010/051654
(87) International publication number: WO 2010/087517

(57) **Abstract**

The object of the present invention is to provide a therapeutic agent or a prophylactic agent for a disease resulting from an abnormal bone metabolism, especially osteoporosis which is more effective than conventional agents. Excellent therapeutic effect or prophylactic effect on a disease resulting from an abnormal bone metabolism, especially osteoporosis, compared with the effect in administration of each compound alone can be obtained by the combination of an N-hydroxybenzamidine derivative represented by Formula (I) or a salt thereof with an activated vitamin D or a prodrug thereof.

## Description

### Technical Field

The present invention relates to a therapeutic agent or a prophylactic agent for a disease resulting from an abnormal bone metabolism, especially a prophylactic agent or a therapeutic agent for osteoporosis, more specifically a therapeutic agent or a prophylactic agent for a disease resulting from an abnormal bone metabolism, especially osteoporosis comprising an N-hydroxybenzamidine derivative represented by Formula (I) or a salt thereof, and an activated vitamin D or a prodrug thereof as active ingredients.

### Background Art

N-hydroxybenzamidine derivatives represented by Formula (I) or salts thereof have a bone resorption preventing effect and an osteogenesis promoting effect, and are expected to be therapeutic agents or prophylactic agents for osteoporosis. The N-hydroxybenzamidine derivatives represented by Formula (I) or salts thereof are disclosed in Patent Publications 1, 2, 3 or 4.

N-hydroxy-4-{5-[4-(5-isopropyl-2-methyl-1,3-thiazol-4-yl)phenoxy]pentoxy}-benzamidine represented by Formula (II) which is one of the N-hydroxybenzamidine derivatives represented by Formula (I) or a salt thereof has a bone resorption preventing effect and an osteogenesis promoting effect, and is expected to be a therapeutic agent or a prophylactic agent for osteoporosis.

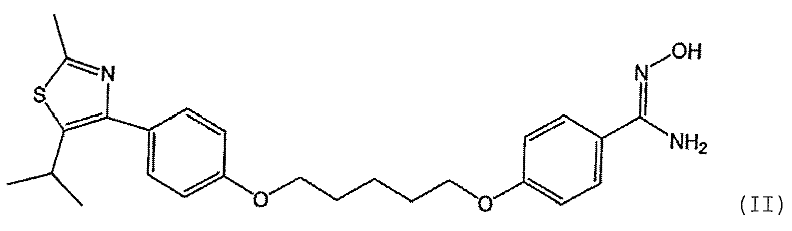

N-hydroxy-4-{5-[4-(5-isopropyl-2-methyl-1,3-thiazol-4-yl)phenoxy]pentoxy}-benzamidine or a salt thereof is disclosed in Patent Publication 4 and Non-patent Literature Publication 1.

An activated vitamin D or a prodrug thereof regulates the bone metabolism as a bone activating agent, and is used as a therapeutic agent for a disease associated with an abnormal calcium metabolism including osteoporosis (Non-patent Literature Publication 2).

There are a large number of available therapeutic agents for osteoporosis. However, the therapeutic effects of these agents in combination are not the same. For example, it is known that effects of PTH (Parathyroid Hormone) and a certain type of a bisphosphonic acid are balanced out in the combination thereof. They are representative osteoporosis therapeutic agents. However, results of a preclinical test and a clinical test that when they were administered in combination, the therapeutic effect thereof was decreased, compared with a case where each agent was administered alone were reported (Non-patent Literature Publications 3 and 4). In addition, regarding the activated vitamin D or a prodrug thereof, in the same manner as the above case of PTH with the bisphosphonic acid, a clinical test result on the combination of the activated vitamin D or a prodrug thereof with vitamin K having a calcium deposition action on the bone that when they were administered in combination, the therapeutic effect thereof was decreased, compared with a case where each agent was administered alone was reported (Non-patent Literature Publication 5).

As is presumed from the above examples, a therapeutic method or a prophylactic method exhibiting a higher effect using conventional or novel osteoporosis therapeutic agents cannot be easily found.
Patent Publication 1 Kohyo (Jpn. Unexamined Patent Publication) No. 2008-509134
Patent Publication 2 Kohyo No. 2009-525321
Patent Publication 3 WO 2009/017346 Pamphlet
Patent Publication 4 Kohyo No. 2004-537549
Non-patent Literature Publication 1 Lee, Sung-Eun, Synthesis and Biological Activity of Natural Products and Designed New Hybrid Compounds for the Treatment of LTB4 Related Disease, the doctoral thesis, the Graduate School, Busan National University, 1999 August
Non-patent Literature Publication 2 Edited by Toshitaka NAKAMURA, Toshio MATSUMOTO, Shigeaki KATO, "Bone Metabolism and Activated Vitamin D -Past and Now, and Future-", Life Science Publishing Co., Ltd., published on September 25, 2006
Non-patent Literature Publication 3 R Samadfam et al, Endocrinology, Vol. 148, No. 6, 2007
Non-patent Literature Publication 4 DM Black et al, The New England Journal of Medicine, Vol. 349, No. 13, 2003
Non-patent Literature Publication 5, Seneki KOBAYASHI, Masataka SHIRAKI, Kunio TAKAOKA, "Problems in Vitamin K Combination Therapy in Osteoporosis Treatment", CLINICAL CALCIUM, Vol. 17, No. 11, 2007

### Disclosure of the Invention

The object of the present invention is to provide a therapeutic agent or a prophylactic agent for a disease resulting from an abnormal bone metabolism, especially osteoporosis which exhibits a higher effect, compared with conventional medicaments. Further, the object of the present invention is to provide a therapeutic method or a prophylactic method for a disease resulting from the abnormal bone metabolism, especially osteoporosis which exhibits a higher effect, compared with conventional therapeutic methods or prophylactic methods.

The inventors of the present application keenly studied active ingredients as therapeutic agents or prophylactic agents for a disease resulting from the abnormal bone metabolism, especially osteoporosis, and as a result, found that a remarkably excellent medicinal effect, compared with the use of each medicament alone can be obtained by the combination of specified medicaments which may be used alone.

Namely, the present invention is described as below.
(1) A therapeutic agent or a prophylactic agent for a disease resulting from the abnormal bone metabolism comprising the following (a) and (b) as active ingredients.
   (a) An N-hydroxybenzamidine derivative represented by the following Formula (I) or a salt thereof.
   (b) An activated vitamin D or a prodrug thereof. 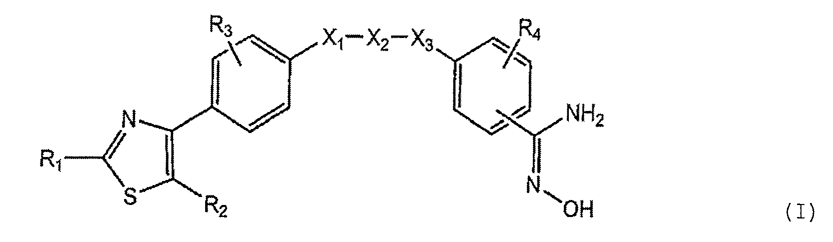 [In the above Formula (I),
      R¹ is hydrogen; C1-C6 alkyl; C3-C6 cycloalkyl; C6-C12 aryl, 5- to 12-membered heteroaryl ring containing 1 to 3 heteroatoms selected from N, O and S; or NR⁵R⁶;
      R² is hydrogen; C1-C6 alkyl; C2-C6 alkenyl; C3-C6 cycloalkyl; amino; C1-3 dialkylamino; C1-3 alkyl substituted by halogen, C3-C6 cycloalkyl, C6-C12 aryl, 5-to 12-membered heteroaryl ring containing 1 to 3 heteroatoms selected from N, O and S, C3-6 heterocycloalkyl containing 1 to 3 heteroatoms selected from N, O and S; C1-3 alkyl substituted by NR⁷R⁸; or N-methylcarbamoyl;
      R³ and R⁴ independently are hydrogen; halogen; hydroxy; C1-6 alkyl which is unsubstituted or is substituted by a halogen; C3-6 cycloalkylamino; C1-6 alkoxy; C1-6 alkanoyloxy; C2-6 alkenyloxy; phenyl-C1-6 alkoxy; phenoxy; C2-6 alkenoyloxy; phenyl-C1-6 alkanoyloxy; C3-6 cycloalkyloxy substituted by carboxy, esterified carboxy or amidated carboxy; or aminooxy;
      R⁵ is hydrogen or C1-C6 alkyl;
      R⁶ is hydrogen; C1-C6 alkyl; C1-3 alkyl substituted by hydroxy, C6-C12 aryl, 5- to 12-membered heteroaryl ring containing 1 to 3 heteroatoms selected from N, O and S, or C3-6 heterocycloalkyl containing 1 to 3 heteroatoms selected from N, O and S;
      R⁷ is hydrogen; C1-C6 alkyl; or C3-C6 cycloalkyl;
      R⁸ is hydrogen; C1-C4 alkyl; C1-3 alkyl substituted by C1-3 alkoxy; or 4-pyridinoyl;
      X¹ is -O-; -S-; -NH-; or -N(C1-6 alkyl)-;
      X² is C3-7 alkylene; C3-7 alkylenecarbonyl; or C1-3 alkylene-phenylene-C1-3 alkylene;
      X³ is -O-; -S-; -NH-; or -N(C1-6 alkyl)-;].
(2) The therapeutic agent or the prophylactic agent for a disease resulting from the abnormal bone metabolism as described in (1), wherein
   1) when R¹ is C1-C6 alkyl,
      R² is C1-C6 alkyl; C2-C6 alkenyl; C3-C6 cycloalkyl; amino; C1-3 dialkylamino; C1-3 alkyl substituted by halogen, C3-C6 cycloalkyl, C6-C12 aryl, 5- to 12-membered heteroaryl ring containing 1 to 3 heteroatoms selected from N, O and S, or C3-6 heterocycloalkyl containing 1 to 3 heteroatoms selected from N, O and S; or C1-3 alkyl substituted by NR⁷R⁸;
   2) when R¹ is C3-C6 cycloalkyl,
      R² is C1-C6 alkyl; or C1-3 alkyl substituted by NR⁷R⁸;
   3) when R¹ is C6-C12 aryl or 5- to 12-membered heteroaryl ring containing 1 to 3 heteroatoms selected from N, O and S,
      R² is C1-C3 alkyl substituted by halogen, C3-C6 cycloalkyl, C6-C12 aryl, 5- to 12-membered heteroaryl ring containing 1 to 3 heteroatoms selected from N, O and S, or C3-6 heterocycloalkyl containing 1 to 3 heteroatoms selected from N, O and S;
   4) when R¹ is NR⁵R⁶,
      R² is C1-C6 alkyl; C3-6 cycloalkyl; C1-C3 alkyl substituted by halogen, C3-C6 cycloalkyl, C6-C12 aryl, 5-to 12-membered heteroaryl ring containing 1 to 3 heteroatoms selected from N, O and S, or C3-6 heterocycloalkyl containing 1 to 3 heteroatoms selected from N, O and S; or N-methylcarbamoyl.
(3) The therapeutic agent or the prophylactic agent for a disease resulting from the abnormal bone metabolism as described in (1) or (2), wherein
   1) when R⁵ is hydrogen,
      R⁶ is hydrogen; or C1-C6 alkyl;
   2) when R⁵ is C1-C6 alkyl,
      R⁶ is C1-C6 alkyl; C1-3 alkyl substituted by a group selected from hydroxy, C6-C12 aryl, 5- to 12-membered heteroaryl ring containing 1 to 3 heteroatoms selected from N, O and S, and C3-6 heterocycloalkyl containing 1 to 3 heteroatoms selected from N, O and S.
(4) The therapeutic agent or the prophylactic agent for a disease resulting from the abnormal bone metabolism as described in any one of (1) to (3), wherein
   1) when R⁷ is hydrogen,
      R⁸ is C1-C4 alkyl; C1-3 alkyl substituted by C1-3 alkoxy or C3-6 heterocycloakyl;
   2) when R⁷ is C1-C6 alkyl,
      R⁸ is C1-C4 alkyl;
   3) when R⁷ is C3-C6 cycloalkyl,
      R⁸ is 4-pyridinoyl.
(5) The therapeutic agent or the prophylactic agent for a disease resulting from the abnormal bone metabolism as described in any one of (1) to (4), wherein R¹ is hydrogen; methyl; ethyl; propyl; isopropyl; cyclohexyl; phenyl; 3-pyridinyl; or NR⁵R⁶.
(6) The therapeutic agent or the prophylactic agent for a disease resulting from the abnormal bone metabolism as described in any one of (1) to (5), wherein
   R² is hydrogen; methyl; ethyl; propyl; isopropyl; butyl; vinyl; cyclopentyl; amino; dimethylamino; methyl or ethyl substituted by chlorine, pentyl, phenyl, 1-imidazolyl, 4-thiomorpholinyl, 4-morpholinyl, or NR⁷R⁸; or methyl or ethyl substituted by N-methylcarbamoyl.
(7) The therapeutic agent or the prophylactic agent for a disease resulting from the abnormal bone metabolism as described in any one of (1) to (6), wherein R³ is hydrogen.
(8) The therapeutic agent or the prophylactic agent for a disease resulting from the abnormal bone metabolism as described in any one of (1) to (7), wherein R⁴ is hydrogen; or fluorine.
(9) The therapeutic agent or the prophylactic agent for a disease resulting from the abnormal bone metabolism as described in any one of (1) to (8), wherein R⁵ is hydrogen; methyl; ethyl; propyl; or isopropyl.
(10) The therapeutic agent or the prophylactic agent for a disease resulting from the abnormal bone metabolism as described in any one of (1) to (9), wherein R⁶ is hydrogen; methyl; ethyl; propyl; isopropyl; or methyl or ethyl substituted by hydroxy, phenyl, 3-pyridinyl or 4-morpholinyl.
(11) The therapeutic agent or the prophylactic agent for a disease resulting from the abnormal bone metabolism as described in any one of (1) to (10), wherein R⁷ is hydrogen; methyl; ethyl or cyclopropyl.
(12) The therapeutic agent or the prophylactic agent for a disease resulting from the abnormal bone metabolism as described in any one of (1) to (11), wherein R⁸ is hydrogen; methyl; ethyl; isopropyl; 3-isopropyloxypropyl; or 4-pyridinoyl.
(13) The therapeutic agent or the prophylactic agent for a disease resulting from the abnormal bone metabolism as described in any one of (1) to (12), wherein
   X¹ is -O-;
   x² is butylene; pentylene; 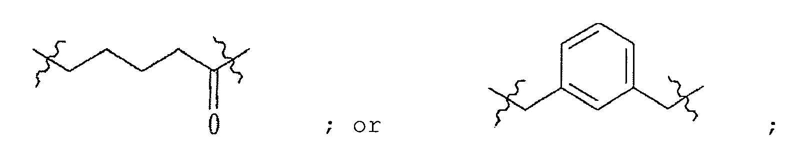
   x³ is -O-; -NH-; or -N(CH₃)- ;.
(14) The therapeutic agent or the prophylactic agent for a disease resulting from the abnormal bone metabolism as described in any one of (1) to (13), wherein
   X¹-X²-X³ is 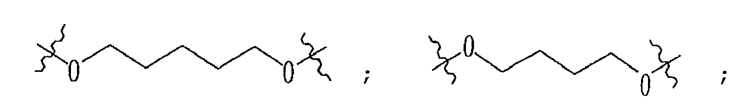 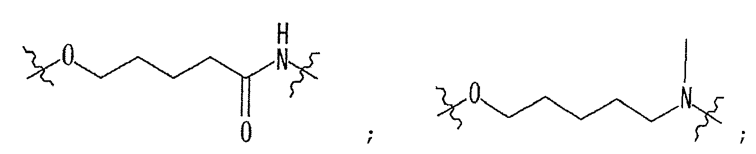 or 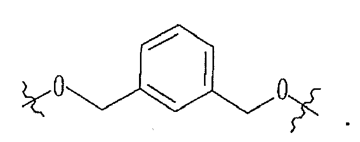
(15) The therapeutic agent or the prophylactic agent for a disease resulting from the abnormal bone metabolism as described in (1), wherein the N-hydroxybenzamidine derivative represented by Formula (I) is any one of the following compounds:
   1) N-hydroxy-4-{5-[4-(5-isopropyl-2-methyl-1,3-thiazol-4-yl)phenoxy]pentoxy}benzamidine
   2) N-hydroxy-4-{5-[4-(5-benzyl-2-(pyridin-3-yl)-1,3-thiazol-4-yl]phenoxy]pentoxy}benzamidine
   3) N-hydroxy-4-{5-[4-(5-(2-chloroethyl)-2-methyl-1,3-thiazol-4-yl)phenoxy]pentoxy}benzamidine
   4) N-hydroxy-4-{5-[4-(5-cyclopentylmethyl-2-ethyl-1,3-thiazol-4-yl)phenoxy]pentoxy}benzamidine
   5) N-hydroxy-4-{5-[4-(5-vinyl-2-methyl-1,3-thiazol-4-yl)phenoxy]pentoxy}benzamidine
   6) N-hydroxy-4-{5-[4-(5-cyclopentylmethyl-2-methylamino-1,3-thiazol-4-yl)phenoxy]pentoxy}benzamidine
   7) N-hydroxy-4-{5-[4-(2-(2-hydroxyethyl)methylamino-5-methyl-1,3-thiazol-4-yl)phenoxy]pentoxy}benzamidine
   8) N-hydroxy-4-{5-[4-(2-benzylmethylamino-5-methyl-1,3-thiazol-4-yl)phenoxy]pentoxy}benzamidine
   9) N-hydroxy-4-{5-[4-(5-methyl-2-(methyl(pyridin-3-ylmethyl)amino)-1,3-thiazol-4-yl)phenoxy]pentoxy}benzamidine
   10) N-hydroxy-4-{5-[4-(2-benzylmethylamino-5-ethyl-1,3-thiazol-4-yl)phenoxy]pentoxy}benzamidine
   11) N-hydroxy-4-{5-[4-(2-dipropylamino-5-ethyl-1,3-thiazol-4-yl)phenoxy]pentoxy}benzamidine
   12) N-hydroxy-4-{5-[4-(5-isopropyl-2-(methyl(pyridin-3-yl-methyl)amino)-1,3-thiazol-4-yl)phenoxy]pentoxy}benzamidine
   13) N-hydroxy-4-{5-[4-(5-butyl-2-(methyl(2-(4-morpholino)ethyl)amino)-1,3-thiazol-4-yl)phenoxy]pentoxy}benzamidine
   14) N-hydroxy-4-{5-[4-(5-cyclopentylmethyl-2-dipropylamino-1,3-thiazol-4-yl)phenoxy]pentoxy}benzamidine
   15) N-hydroxy-4-{5-[4-(5-cyclopentyl-2-(methyl(2-(4-morpholino)ethyl)amino)-1,3-thiazol-4-yl)phenoxy]pentoxy}benzamidine
   16) N-hydroxy-4-{5-[4-(5-isopropyl-2-dipropylamino-1,3-thiazol-4-yl)phenoxy]pentoxy}benzamidine
   17) N-hydroxy-4-{5-[4-(5-isopropyl-2-dimethylamino-1,3-thiazol-4-yl)phenoxy]pentoxy}benzamidine
   18) N-hydroxy-4-{5-[4-(5-isopropyl-2-methyl-1,3-thiazol-4-yl)phenoxy]pentanoylamino}benzamidine
   19) N-hydroxy-4-{5-[4-(5-isopropyl-2-methyl-1,3-thiazol-4-yl)phenoxy]pentylmethylamino}benzamidine
   20) N-hydroxy-2-fluoro-4-{5-[4-(5-isopropyl-2-methyl-1,3-thiazol-4-yl)phenoxy]pentoxy}benzamidine
   21) N-hydroxy-4-{4-[4-(2-cyclohexyl-5-ethyl-1,3-thiazol-4-yl)phenoxy]butoxy}benzamidine
   22) N-hydroxy-4-{3-[4-(5-isopropyl-2-methyl-1,3-thiazol-4-yl)phenoxymethyl]benzyloxy}benzamidine
   23) N-hydroxy-4-{5-[4-(5-(2-(imidazol-1-yl)ethyl)-2-methyl-1,3-thiazol-4-yl)phenoxy]pentoxy}benzamidine
   24) N-hydroxy-4-{5-[4-(5-(2-(isopropylamino)ethyl)-2-methyl-1,3-thiazol-4-yl)phenoxy]pentoxy}benzamidine
   25) N-hydroxy-4-{5-[4-(5-(2-(3-(isopropoxy)propylamino)ethyl)-2-methyl-1,3-thiazol-4-yl)phenoxy]pentoxy}benzamidine
   26) N-hydroxy-4-{5-[4-(5-(2-diethylaminoethyl)-2-methyl-1,3-thiazol-4-yl)phenoxy]pentoxy}benzamidine
   27) N-hydroxy-4-{5-[4-(2-isopropyl-5-(2-(thiomorpholin-4-yl)ethyl)-1,3-thiazol-4-yl)phenoxy]pentoxy}benzamidine
   28) N-hydroxy-4-{5-[4-(2-cyclohexyl-5-(2-(dimethylamino)ethyl)-1,3-thiazol-4-yl)phenoxy]pentoxy}benzamidine
   29) N-hydroxy-4-{5-[4-(2-dimethylamino-5-(2-(morpholin-4-yl)ethyl)-1,3-thiazol-4-yl)phenoxy]pentoxy}benzamidine
   30) N-hydroxy-4-{5-[4-(5-(2-(cyclopropyl(pyridin-4-carbonyl)amino)ethyl)-2-isopropyl-1,3-thiazol-4-yl)phenoxy]pentoxy}benzamidine
   31) N-hydroxy-4-{5-[4-(2-amino-5-(N-methylcarbamoyl)-1,3-thiazol-4-yl)phenoxy]pentoxy}benzamidine
   32) N-hydroxy-4-{5-[4-(5-(imidazol-1-ylmethyl)-2-methyl-1,3-thiazol-4-yl)phenoxy]pentoxy}benzamidine
   33) N-hydroxy-4-{5-[4-(2-ethylmethylamino-5-(morpholin-4-ylmethyl)-1,3-thiazol-4-yl)phenoxy]pentoxy}benzamidine
   34) N-hydroxy-4-{5-[4-(2-(methyl(2-morpholin-4-yl)ethyl)amino)-5-(thiomorpholin-4-yl)-1,3-thiazol-4-yl)phenoxy]pentoxy}benzamidine
   35) N-hydroxy-4-{5-[4-(5-dimethylamino-2-methyl-1,3-thiazol-4-yl)phenoxy]pentoxy}benzamidine.
(16) The therapeutic agent or the prophylactic agent for a disease resulting from the abnormal bone metabolism as described in (1), wherein the N-hydroxybenzamidine derivative represented by Formula (I) is N-hydroxy-4-{5-[4-(5-isopropyl-2-methyl-1,3-thiazol-4-yl) phenoxy]pentoxy}benzamidine.
(17) The therapeutic agent or the prophylactic agent for a disease resulting from the abnormal bone metabolism as described in any one of (1) to (16), wherein the disease resulting from the abnormal bone metabolism is a disease resulting from abnormal bone resorption.
(18) The therapeutic agent or the prophylactic agent for a disease resulting from the abnormal bone metabolism as described in any one of (1) to (16), wherein the disease resulting from the abnormal bone metabolism is osteodystrophy.
(19) The therapeutic agent or the prophylactic agent for a disease resulting from the abnormal bone metabolism as described in any one of (1) to (16), wherein the disease resulting from the abnormal bone metabolism is osteoporosis.
(20) The therapeutic agent or the prophylactic agent for a disease resulting from the abnormal bone metabolism as described in any one of (1) to (19), wherein (b) is 1α,25-dihydroxyvitamin D₃, 1α,24R-dihydroxyvitamin D₃, or 1α-hydroxyvitamin D₃.
(21) The therapeutic agent or the prophylactic agent for a disease resulting from the abnormal bone metabolism as described in any one of (1) to (19), wherein (b) is 1α,25-dihydroxyvitamin D₃ or 1α-hydroxyvitamin D₃.
(22) The therapeutic agent or the prophylactic agent for a disease resulting from the abnormal bone metabolism as described in any one of (1) to (21), wherein (a) and (b) form a drug.combination.
(23) The therapeutic agent or the prophylactic agent for a disease resulting from the abnormal bone metabolism as described in any one of (1) to (21), wherein (a) and (b) each is an independent single agent.
(24) The therapeutic agent or the prophylactic agent for a disease resulting from the abnormal bone metabolism as described in any one of (1) to (21), which is a kit comprising (a) and (b).

Further, the present invention relates to a therapeutic method or a prophylactic method for a disease resulting from an abnormal bone metabolism, especially osteoporosis comprising administering an agent comprising the above (a) and (b) as active ingredients.

### Effect of the Invention

The present invention relates to a therapeutic agent or a prophylactic agent for a disease resulting from an abnormal bone metabolism, especially osteoporosis comprising both an N-hydroxybenzamidine derivative represented by Formula (I) or a salt thereof, and an activated vitamin D or a prodrug thereof as active ingredients. Further, the present invention relates to a therapeutic method or a prophylactic method for a disease resulting from an abnormal bone metabolism, especially osteoporosis comprising administering these agents. Compared with a case where the N-hydroxybenzamidine derivative represented by Formula (I) or a salt thereof, or the activated vitamin D or a prodrug thereof is used alone, remarkably significant therapeutic effect or prophylactic effect can be obtained by administering both the active ingredients in combination.

### Brief Description of Drawings

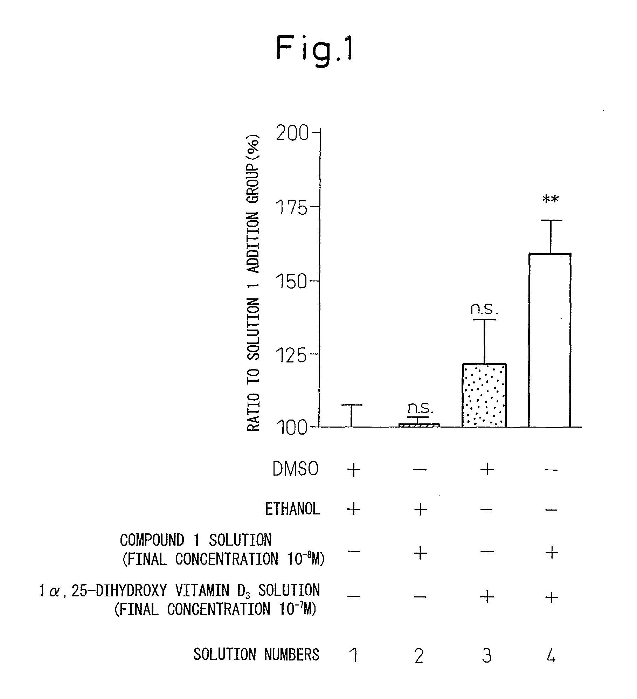

FIG 1: A graph showing a cell growth-promoting effect of the combination of two of an N-hydroxy-4-{5-[4-(5-isopropyl-2-methyl-1,3-thiazol-4-yl) phenoxy]pentoxy}-benzamidine (referred to as Compound 1, hereinafter) solution, a 1α,25-dihydroxyvitamin D₃ solution, dimethylsulfoxide (referred to as DMSO, hereinafter) (a solvent used to prepare a Compound 1 solution), and ethanol (a solvent used to prepare a 1α,25-dihydroxyvitamin D₃ solution) on a rat osteosarcoma cell strain (ROS17/2.8 cells) stimulated by the combination.

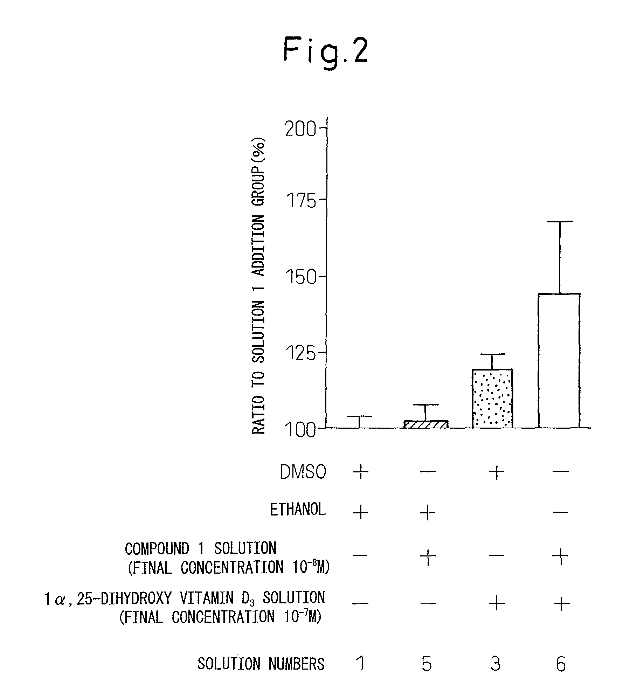

FIG 2: A graph showing a cell growth-promoting effect of the combination of two of an N-hydroxy-4-5-[4-(2-dipropylamino-5-ethyl-1,3-thiazol-4-yl)phenoxy]pentoxy}benzamidine (referred to as Compound 11, hereinafter) solution, a 1α,25-dihydroxyvitamin D₃ solution, dimethylsulfoxide (referred to as DMSO, hereinafter) (a solvent used to prepare a Compound 11 solution), and ethanol (a solvent used to prepare a 1α,25-dihydroxyvitamin D₃ solution) on a rat osteosarcoma cell strain (ROS17/2.8 cells) stimulated by the combination.

### Mode for Carrying Out the Invention

The N-hydroxybenzamidine derivative represented by Formula (I) or a salt thereof used in the present invention has excellent bone resorption preventing effect and osteogenesis promoting effect, and is expected to be a therapeutic agent or a prophylactic agent for a disease resulting from an abnormal bone metabolism, especially osteoporosis.

The N-hydroxybenzamidine derivative represented by Formula (I) is described in Patent Publication 1, 2 or 3 and Non-patent Literature Publication 1, and can be produced by referring to the descriptions of these patent publications.

The N-hydroxybenzamidine derivative represented by Formula (I) in the present invention is a compound below. 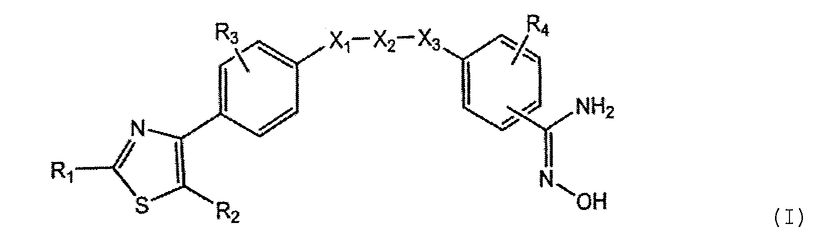 [In the above Formula (I),
R¹ is hydrogen; C1-C6 alkyl; C3-C6 cycloalkyl; C6-C12 aryl; 5- to 12-membered heteroaryl ring containing 1 to 3 heteroatoms selected from N, O and S; or NR⁵R⁶;
R² is hydrogen; C1-C6 alkyl; C2-C6 alkenyl; C3-C6 cycloalkyl; amino; C1-3 dialkylamino; C1-3 alkyl substituted by halogen, C3-C6 cycloalkyl, C6-C12 aryl, 5-to 12-membered heteroaryl ring containing 1 to 3 heteroatoms selected from N, O and S, C3-6 heterocycloalkyl containing 1 to 3 heteroatoms selected from N, O and S; C1-3 alkyl substituted by NR⁷R⁸; or N-methylcarbamoyl;
R³ and R⁴ independently are hydrogen; halogen; hydroxy; C1-6 alkyl which is unsubstituted or is substituted by a halogen; C3-6 cycloalkylamino; C1-6 alkoxy; C1-6 alkanoyloxy; C2-6 alkenyloxy; phenyl-C1-6 alkoxy; phenoxy; C2-6 alkenoyloxy; phenyl-C1-6 alkanoyloxy; C3-6 cycloalkyloxy substituted by carboxy, esterified carboxy or amidated carboxy; or aminooxy;
R⁵ is hydrogen or C1-C6 alkyl;
R⁶ is hydrogen; C1-C6 alkyl; C1-3 alkyl substituted by hydroxy, C6-C12 aryl, 5- to 12-membered heteroaryl ring containing 1 to 3 heteroatoms selected from N, O and S, or C3-6 heterocycloalkyl containing 1 to 3 heteroatoms selected from N, O and S;
R⁷ is hydrogen; C1-C6 alkyl; or C3-C6 cycloalkyl;
R⁸ is hydrogen; C1-C4 alkyl; C1-3 alkyl substituted by C1-3 alkoxy; or 4-pyridinoyl;
X¹ is -O-; -S-; -NH-; or -N(C1-6 alkyl)-;
X² is C3-7 alkylene; C3-7 alkylenecarbonyl; or C1-3 alkylene-phenylene-C1-3 alkylene;
X³ is -O-; -S-; -NH-; or -N(C1-6 alkyl)-].

In addition, R¹ is preferably hydrogen; methyl; ethyl; propyl; isopropyl; cyclohexyl; phenyl; 3-pyridinyl; or NR⁵R⁶, more preferably, methyl; ethyl; isopropyl; cyclohexyl; 3- pyridinyl; or NR⁵R⁶.

R² is preferably hydrogen; methyl; ethyl; propyl; isopropyl; butyl; vinyl; cyclopentyl; amino; dimethylamino; methyl or ethyl substituted by chlorine, pentyl, phenyl, 1-imidazolyl, 4-thiomorpholinyl, 4-morpholinyl, or NR⁵R⁶; or methyl or ethyl substituted by N-methylcarbamoyl, more preferably, methyl; ethyl; isopropyl; butyl; vinyl; cyclopentyl; dimethylamino; methyl or ethyl substituted by chlorine, pentyl, phenyl, 1-imidazolyl, 4-thiomorpholinyl, 4-morpholnyl, or NR⁵R⁶, or methyl or ethyl substituted by N-methylcarbamoyl.

R³ is preferably hydrogen.

R⁴ is preferably hydrogen; or fluorine.

R⁵ is preferably hydrogen; methyl; ethyl; propyl; or isopropyl, more preferably hydrogen; methyl; ethyl; or propyl.

R⁶ is preferably hydrogen; methyl; ethyl; propyl; isopropyl; or methyl or ethyl substituted by hydroxy, phenyl, 3-pyridinyl or 4-morpholinyl, more preferably hydrogen; methyl; ethyl; propyl; or methyl or ethyl substituted by hydroxy, phenyl, 3-pyridinyl or 4-morpholinyl.

R⁷ is preferably hydrogen; methyl; ethyl; or cyclopropyl.

R⁸ is preferably hydrogen; methyl; ethyl; isopropyl; 3-isopropyloxypropyl; or 4-pyridinoyl.

It is preferable that X¹ is -O-;
X² is butylene; pentylene; 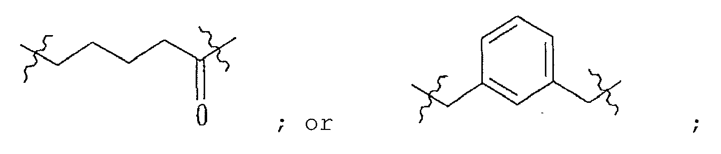 x³ is preferably -O-; -NH-; or N(CH₃)-;.

Further, X¹-X²-X³ is 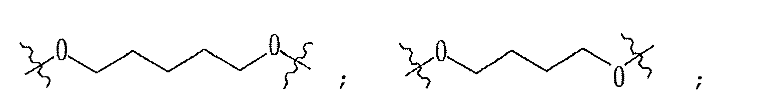 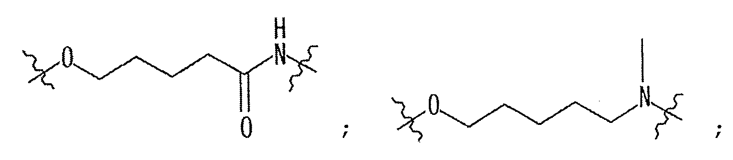 or

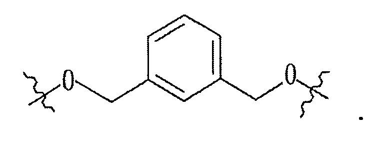

In particular, the derivatives described in Table 1 or salts thereof are preferable as the N-hydroxybenzamidine derivatives represented by Formula (I). Among them, N-hydroxy-4-5-[4-(5-isopropyl-2-methyl-1,3-thiazol-4-yl)
phenoxy]pentoxy-benzamidine of Compound No. 1 is especially preferable.

These have excellent bone resorption preventing effect and osteogenesis promoting effect, and are expected to be therapeutic agents or prophylactic agents for a disease resulting from the abnormal bone metabolism, especially osteoporosis, and can be used in combination with the activated vitamin D or a prodrug thereof. An agent comprising both compounds as active ingredients is effective as a therapeutic agent or prophylactic agent for a disease resulting from the abnormal bone metabolism, especially osteoporosi.

**Table 1**

| Compounc No. | Structure | Compound Name |
|---|---|---|
| 1 | 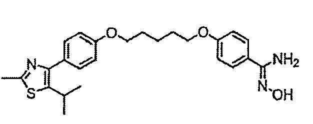 | N-hydroxy-4-{5-[4-(5-isopropyl-2-methyl-1,3-thiazol-4-yl)phenoxy] pentoxy}benzamidine |
| 2 | 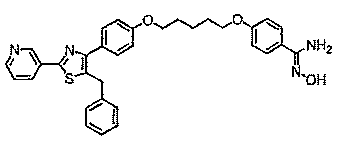 | N-hydroxy-4-{5-[4-(5-benzyl-2-(pyridin-3-yl)-1,3-thiazol-4-yl]phenoxy]pentoxy}benzamidine |
| 3 | 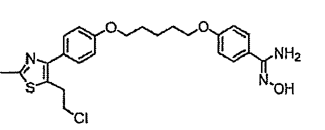 | N-hydroxy-4-{5-[4-(5-(2-chloroethyl)-2-methyl-1,3-thiazol-4-yl) phenoxy]pentoxy} benzamidine |
| 4 | 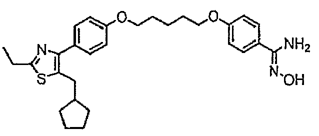 | N-hydroxy-4-{5-[4-(5-cyclopentylmethyl-2-ethyl-1,3-thiazol-4-yl)phenoxy]pentoxy} benzamidine |
| 5 | 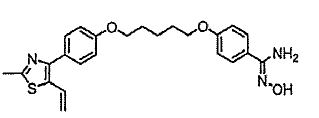 | N-hydroxy-4-{5-[4-(5-vinyl-2-methyl-1,3-thiazol-4-yl) phenoxy]pentoxy}benzamidine |
| 6 | 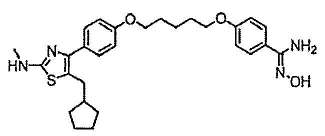 | N-hydroxy-4-{5-[4-(5-cyclopentylmethyl-2-methylamino-1,3-thiazol-4-yl) phenoxy]pentoxy}benzamidine |
| 7 | 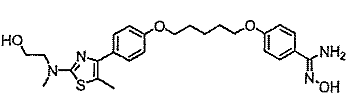 | N-hydroxy-4-{5-[4-(2-(2-hydroxyethyl)methylamino-5-methyl-1,3-thiazol-4-yl) phenoxy]pentoxy}benzamidine |
| 8 | 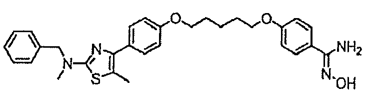 | N-hydroxy-4-{5-[4-(2-benzylmethylamino-5-methyl-1,3-thiazol-4-yl)phenoxy] pentoxy}benzamidine |
| 9 | 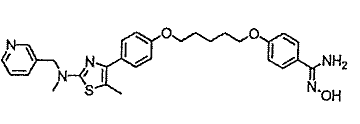 | N-hydroxy-4-{5-[4-(5-methyl-2-(methyl(pyridin-3-ylmethyl) amino)-1,3-thiazol-4-yl) phenoxy]pentoxy}benzamidine |
| 10 | 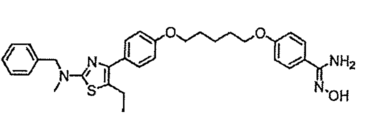 | N-hydroxy-4-{5-[4-(2-benzylmethylamino-5-ethyl-1,3-thiazol-4-yl)phenoxy]pentoxy} benzamidine |
| 11 | 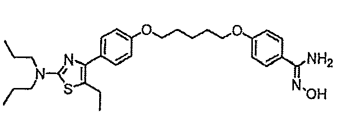 | N-hydroxy-4-{5-[4-(2-dipropylamino-5-ethyl-1,3-thiazol-4-yl)phenoxy]pentoxy} benzamidine |
| 12 | 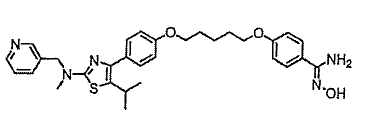 | N-hydroxy-4-{5-[4-(5-isopropyl-2-(methyl(pyridin-3-yl-methyl)amino)-1,3-thiazol-4-yl)phenoxy]pentoxy} benzamidine |
| 13 | 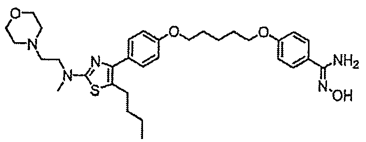 | N-hydroxy-4-{5-[4-(5-butyl-2-(methyl(2-(4-morpholino)ethyl) amino)-1,3-thiazol-4-yl) phenoxy]pentoxy}benzamidine |
| 14 | 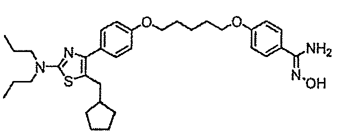 | N-hydroxy-4-{5-[4-(5-cyclopentylmethyl-2-dipropylamino-1,3-thiazol-4-yl)phenoxy]pentoxy}benzamidine |
| 15 | 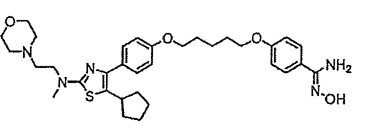 | N-hydroxy-4-{5-[4-(5-cyclopentyl-2-(methyl(2-(4-morpholino)ethyl)amino)-1,3-thiazol-4-yl)phenoxy] pentoxy}benzamidine |
| 16 | 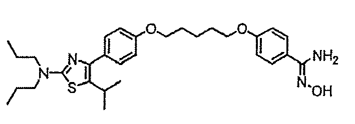 | N-hydroxy-4-{5-[4-(5-isopropyl-2-dipropylamino-1,3-thiazol-4-yl)phenoxy]pentoxy} benzamidine |
| 17 | 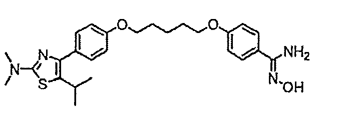 | N-hydroxy-4-{5-[4-(5-isopropyl -2-dimethylamino-1,3-thiazol-4-yl)phenoxy]pentoxy} benzamidine |
| 18 | 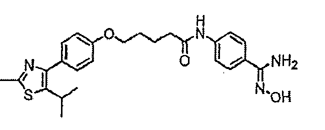 | N-hydroxy-4-{5-[4-(5-isopropyl-2-methyl-1,3-thiazol-4-yl)phenoxy] pentanoylamino}benzamidine |
| 19 | 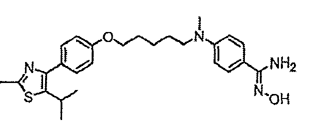 | N-hydroxy-4-{5-[4-(5-isopropyl-2-methyl-1,3-thiazol-4-yl)phenoxy] pentylmethylamino}benzamidine |
| 20 | 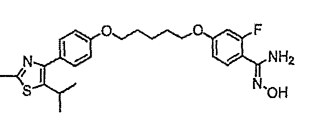 | N-hydroxy-2-fluoro-4-{5-[4-(5-isopropyl-2-methyl-1,3-thiazol-4-yl)phenoxy] pentoxy}benzamidine |
| 21 | 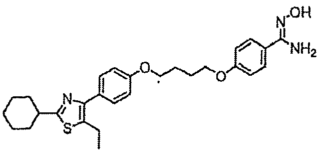 | N-hydroxy-4-{4-[4-(2-cyclohexyl-5-ethyl-1,3-thiazol-4-yl)phenoxy] butoxy}benzamidine |
| 22 | 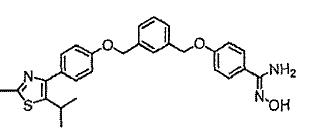 | N-hydroxy-4-{3-[4-(5-isopropyl-2-methyl-1,3-thiazol-4-yl)phenoxymethyl] benzyloxy}benzamidine |
| 23 | 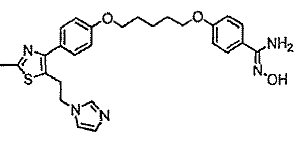 | N-hydroxy-4-{5-[4-(5-(2-(imidazol-1-yl)ethyl)-2-methyl-1,3-thiazol-4-yl) phenoxy]pentoxy}benzamidine |
| 24 | 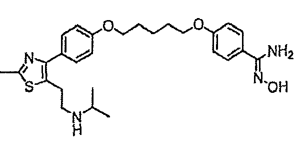 | N-hydroxy-4-{5-[4-(5-(2-(isopropylamino)ethyl)-2-methyl-1,3-thiazol-4-yl) phenoxy]pentoxy}benzamidine |
| 25 | 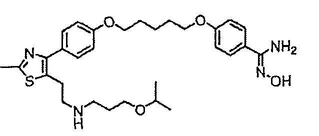 | N-hydroxy-4-{5-[4-(5-(2-(3-(isopropoxy)propylamino)ethyl) -2-methyl-1,3-thiazol-4-yl) phenoxy]pentoxy}benzamidine |
| 26 | 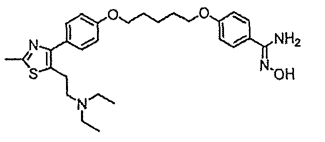 | N-hydroxy-4-{5-[4-(5-(2-diethylaminoethyl)-2-methyl-1,3-thiazol-4-yl)phenoxy] pentoxy}benzamidine |
| 27 | 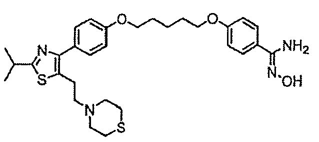 | N-hydroxy-4-{5-[4-(2-isopropyl-5-(2-(thiomorpholin-4-yl)ethyl)-1,3-thiazol-4-yl) phenoxy]pentoxy}benzamidine |
| 28 | 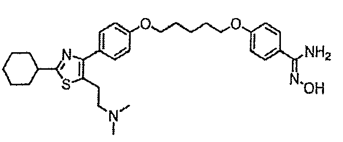 | N-hydroxy-4-{5-[4-(2-cyclohexyl-5-(2-(dimethylamino)ethyl)-1,3-thiazol-4-yl)phenoxy]pentoxy} benzamidine |
| 29 | 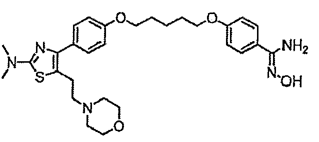 | N-hydroxy-4-{5-[4-(2-dimethylamino-5-(2-(morpholin-4-yl)ethyl)-1,3-thiazol-4-yl) phenoxy]pentoxy}benzamidine |
| 30 | 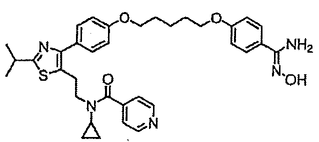 | N-hydroxy-4-{5-[4-(5-(2-(cyclopropyl(pyridin-4-carbonyl)amino) ethyl}-2-isopropyl-1,3-thiazol-4-yl)phenoxy]pentoxy} benzamidine |
| 31 | 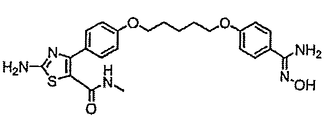 | N-hydroxy-4-{5-[4-(2-amino-5-(N-methylcarbamoyl)-1,3-thiazol-4-yl)phenoxy]pentoxy} benzamidine |
| 32 | 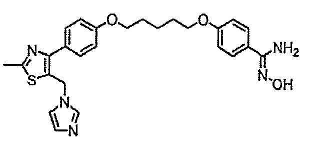 | N-hydroxy-4-{5-[4-(5-(imidazol-1-ylmethyl)-2-methyl-1,3-thiazol-4-yl] phenoxy}pentoxy}benzamidine |
| 33 | 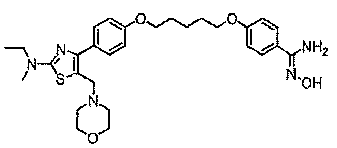 | N-hydroxy-4-{5-[4-(2-ethylmethylamino-5-(morpholin-4-ylmethyl)-1,3-thiazol-4-yl) phenoxy]pentoxy}benzamidine |
| 34 | 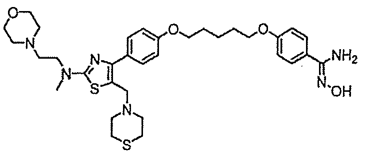 | N-hydroxy-4-{5-{4-(2-(methyl(2-(morpholin-4-yl) ethyl)amino)-5-(thiomorpholin-4-yl)-1,3-thiazol-4-yl)phenoxy]pentoxy} benzamidine |
| 35 | 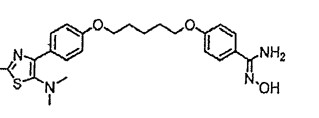 | N-hydroxy-4-{5-[4-(5-dimethylamino-2-methyl-1,3-thiazol-4-yl)phenoxy]pentoxy} benzamidine |

Benzamidine derivatives which are dehydroxy compounds of N-hydroxybenzamidine derivatives represented by Formula (I) or salts thereof, or salts thereof exhibit excellent bone resorption preventing effect and osteogenesis promoting effect, are expected to be therapeutic agents or a prophylactic agents for a disease resulting from the abnormal bone metabolism, especially osteoporosis, and may be used with an activated vitamin D or a prodrug thereof. An agent comprising both compounds as active ingredients is effective as a therapeutic agent or a prophylactic agent for a disease resulting from the abnormal bone metabolism, especially osteoporosis.

Pharmaceutically acceptable salts using inorganic acids (hydrochloric acid, bromic acid, sulfuric acid, and phosphoric acid), and organic acids (citric acid, acetic acid, lactic acid, tartaric acid, fumaric acid, formic acid, propionic acid, oxalic acid, trifluoroacetic acid, methane sulfonic acid, ethane sulfonic acid, benzoic acid, maleic acid, gluconic acid, glycolic acid, succinic acid, 4-toluenesulfonic acid, galacturonic acid, embonic acid, glutamic acid or aspartic acid) are exemplified as salts of the N-hydroxybenzamidine derivatives represented by Formula (I). In the present invention, hydrochloric acid is preferably used as the inorganic acid, and methane sulfonic acid or ethane sulfonic acid is preferably used as the organic acid.

The dosage of the N-hydroxybenzamidine derivative represented by Formula (I) or a salt thereof is an amount effective to prevent or treat a disease resulting from the abnormal bone metabolism, especially osteoporosis. The dosage cannot be generally selected because it is selected, depending on the age or weight of a patient, the type of combination therapy, frequency of the treatment, the type of desired effect, an administration method or the like. However, when it is used as a general therapeutic agent or prophylactic agent, the dosage thereof may be a normally administered amount.

The activated vitamin D or a prodrug thereof used in the present invention has affinity with a vitamin D receptor, and may be any compound which exhibits a physiological action resulting from the affinity. Examples of the activated vitamin D include 1α,25-dihydroxyvitamin D₃ (calcitriol), 1α, 24R-dihydroxyvitamin D₃ (tacalcitol), 2β-(3-hydroxypropoxy)- 1α,25-dihydroxyvitamih D3 (eldecalcitol, ED-71), 1α, 25-dihydroxy-2-methylen-19-norvitamin D₃ (2MD), 1α,25-dihydroxy-22-oxavitamin D₃ (maxacalcitol, OCT), 1α, 24S-dihydroxy-22,23:26,27-tetradehydro-vitamin D₃ (calcipotriol, MC903), 1α,25-dihydroxy-26,26,26,27,27,27-hexafluorovitamin D₃ (farecalcitriol), (23E)-1α-fluroro-25-hydroxy-16,17,23,24-tetrahydro-26,27-bishomo-20-epivitamin D₃ (elocalcitol, BXL-628), 19-nor-1α,25-dihydroxyvitamin D₂ (paricalcitol) and the like. Examples of the prodrug of the activated vitamin D include 1α-hydroxyvitamin D₃ (alphacalcidol), 1α-hydroxyvitamin D₂ (doxacalciferol) and the like.

Alphacalcidol is preferable as the activated vitamin D or a prodrug thereof in the present invention.

In the combination of the N-hydroxybenzamidine derivative represented by Formula (I) or a salt thereof with the activated vitamin D or a prodrug thereof in the present invention, one type of each compound or two or more types of each compound may be mixed in an optional ratio to be used. It is preferable that one type of each compound selected from the above compounds is used in combination.

"Prodrug" used in the specification of the present application means any compounds which are subjected to hydrolysis, being metabolized, derivatization or the like in vivo to be formed into active compounds which exhibit desired activity. In addition, these prodrugs may be those which are converted into active compounds under physiological conditions. Further, regarding vitamin D, as observed in 1α-hydroxyvitamin D₃ (alphacalcidol), it is known that position 25 of a vitamin D is subjected to hydroxylation in vivo, especially in the liver, and the compound is converted into 1α,25-dihydroxyvitamin D₃ of an active compound which exhibits an action on the bone metabolism as an activated vitamin D. The prodrug includes compounds before being subjected to hydroxylation such as 1α-hydroxyvitamin D₃ (published in 1990, "Development of Pharmaceutical Products", Vol. 7, "Molecular Design", p185-186, Hirokawa Shoten, 1990).

Certain types of the activated vitamin D or prodrugs thereof form salts. Pharmaceutically acceptable salts using inorganic acids (hydrochloric acid, bromic acid, sulfuric acid, and phosphoric acid), organic acids (citric acid, acetic acid, lactic acid, tartaric acid, fumaric acid, formic acid, propionic acid, oxalic acid, trifluoroacetic acid, methane sulfonic acid, benzoic acid, maleic acid, gluconic acid, glycolic acid, succinic acid, 4-toluenesulfonic acid, galacturonic acid, embonic acid, glutamic acid or asparagic acid), or inorganic bases (salts of alkaline metals such as sodium, potassium and the like, salts of alkaline earth metals such as magnesium, calcium and the like, salts of metals such as aluminum, zinc and the like) or organic bases (ammonia, triethylamine, diethylamine, ethylenediamine, propanediamine, pyrrolidine, piperidine, piperazine, pyridine, lysine, choline, ethanolamine, diethanolamine, N,N-dimethylethanolamine, 4-hydroxypiperidine, glucosamine, N-methylglucamine and the like) are exemplified as salts of the activated vitamin D or a prodrug thereof.

Regarding the therapeutic agent or the prophylactic agent for osteoporosis of the present invention, an agent having a higher effect to increase the bone density and/or bone strength than therapeutic agents for a disease resulting from the abnormal bone metabolism, especially osteoporosis can be provided by the combination of the two active ingredients, i.e., the activated vitamin D or a prodrug thereof and the N-hydroxybenzamidine derivative represented by Formula (I) or a salt thereof. Further, the agent of the present invention has less toxicity, and is excellent in stability. The amount of each active ingredient can be reduced by the combination thereof, compared with a case where said each active ingredient is administered alone.

The dosage of the activated vitamin D or a prodrug thereof is an amount effective to treat or prevent a disease resulting from the abnormal bone metabolism, especially osteoporosis. The dosage cannot be generally selected because it is selected, depending on the age or weight of a patient, the type of combination therapy, frequency of the treatment, the type of desired effect, an administration method or the like. However, when it is used as a general therapeutic agent or prophylactic agent, the dosage thereof may be a normally administered amount.

The therapeutic agent or the prophylactic agent, or the therapeutic method or the prophylactic method for a disease resulting from the abnormal bone metabolism, especially osteoporosis of the present invention may any agent or method which includes the N-hydroxybenzamidine derivative represented by Formula (I) or a salt thereof, and the activated vitamin D or a prodrug thereof as active ingredients. Both the active ingredients had been mixed, and may be concomitantly administered, or each active ingredient may be separately administered at the same time, consecutively or intermittently. If administration does not occur at the same time, both the active ingredients may be alternatively administered, or after one active ingredient is continuously administered, the other active ingredient may be administered.

The agent of the present invention may be any agent comprising the N-hydroxybenzamidine derivative represented by Formula (I) or a salt thereof, and the activated vitamin D or a prodrug thereof as active ingredients, and may have any form. For example, a drug combination comprising both the active ingredients may be formulated, or each active ingredient may be formulated into a single agent. The drug combination means here those in which two or more active ingredients are contained in one formulation. The single agent means those in which one active ingredient is contained in one formulation. If the agent has a single-agent form, and a plurality of compounds of the activated vitamin D or prodrugs thereof are used, each compound may be used in the form of a single agent or a drug combination. However, the mode using each compound in the form of a single agent is preferable.

The therapeutic agent or the prophylactic agent, or the therapeutic method or the prophylactic method in the case where both the active agents are in the form of a single agent in the present invention means an agent or a method in which a single agent which may be used alone is combined to be used. Therefore, agents comprising each active ingredient may have a different dosage form. For example, the forms of both the agents may be solid or liquid, or solid and liquid. The forms are not particularly limited. When both the active ingredients are a single agent, the dosage form may be a kit which is a set of both the agents. Blister package in which both the agents to be administered for a specific period of time predetermined, based on a series of an administration schedule (for example, a period of one week or longer) are wrapped in one sheet is exemplified as a representative kit form. Other dosage forms are prepared by packing both the agents in the same package by PTP or the like at the end of the production process of the agents, or both the agents may be put in the same bag when dispensed at hospital, pharmacy or the like, and are not particularly limited.

Regarding a drug combination, for example, each active ingredient in an appropriate amount with which the effect of said each active ingredient can be exhibited is combined to produce dosage forms such as tablets, capsules, liquid and the like. The timing at which both the active ingredients are combined to prepare a drug combination may be in the production process of the dosage form of a drug combination or immediately before administration of the dosage form. When the drug combination is prepared in the production process, for example, an appropriate amount of each active ingredient may be mixed to be formulated or packed. A formulation method includes, for example, mixing of each agent or laminating thereof, and is not particularly limited. When a drug combination is prepared immediately before administration, for example, each active ingredient is stored in an independent state until administration, and agents in liquid form are mixed at the time of administration, a solid agent such as a tablet, a pill, a granule, a powder, a capsule or the like is dissolved in a liquid agent, or solid agents such as granules, a powder and the like are mixed to each other. The method for mixing the agents immediately before administration may be performed by hands, or using a package or the like by which both the agents are easily mixed by cutting, pulling, splitting or withdrawing the package. The forms of the drug combination include tablets, pills, granules, powders, liquid, suspension, syrup, capsules and the like.

In addition, the therapeutic agent or the prophylactic agent for a disease resulting from the abnormal bone metabolism, especially osteoporosis may be administered on consecutive days or in an intermittent manner. The number of administrations per day may be one or two to three times. The number of administrations when both the active ingredients are in the form of a single agent is such that the number of administration of each single agent may be the same or different. Further, when both the agents are in the form of a single agent, a general number of administrations of each agent may be combined. As described above, when the number of administrations of each of both the agents is selected to be different, it is expected that it would be more convenient if a form of a kit such as a blister package or the like is selected.

The active ingredients per se, i.e., the N-hydroxybenzamidine derivative represented by Formula (I) or a salt thereof, and the activated vitamin D or a prodrug thereof or those with appropriate additives explained below can be formulated by a conventional method. Specific dosage form thereof include oral preparations such as soft capsules, hard capsules, tablets, syrup and the like, injections and external preparations.

The formulation comprising the active ingredients of the present invention is prepared using additives generally used in formulation processes. The additives for solid formulations include excipients such as lactose, sucrose, glucose, corn starch, potato starch, crystalline cellulose, light anhydrous silicic acid, synthetic aluminum silicate, magnesium aluminometasilicate, calcium hydrogen phosphate and the like; binders such as crystalline cellulose, carboxymethylcellulose, hydroxypropylcellulose, carboxymethylcellulose sodium, polyvinyl pyrrolidone and the like; disintegrating agents such as starch, carboxymethylcellulose sodium, carboxymethylcellulose calcium, croscarmellose sodium, carboxymethyl starch sodium and the like; lubricants such as talc, stearic acid and the like; coating agents such as hydroxymethylpropylcellulose, hydroxypropylmethylcellulose phthalate, ethylcellulose and the like; and colorants; additives for semi-solid formulations include bases such as white vaseline and the like, and additives for liquid formulations include solvents such as ethanol and the like, solubilizing agents such as ethanol and the like, preservatives such as paraoxybenzoic acid esters and the like, tonicity agents such as glucose and the like, buffering agents such as citric acid and the like, antioxidants such as L-ascorbic acid and the like, chelating agents such as EDTA and the like, and suspending agents and emulsifying agents such as polysorbate 80 and the like.

The disease resulting from the abnormal bone metabolism in the present invention is not limited to osteoporosis, but includes diseases resulting from abnormal bone resorption and diseases resulting from the reduction of osteogenesis in the bone metabolism. Osteogenesis imperfect, rickets, osteomalacia, diabetic osteoporosis and glucocorticoid-induced osteoporosis and the like which are categorized into secondary osteoporosis are exemplified as the diseases resulting from the reduction of osteogenesis. In addition, diseases in which a significant therapeutic effect or prophylactic effect can be obtained by promoting osteogenesis include bone fracture including delayed union of fractures (intractable fracture and pseudarthrosis). The diseases resulting from the abnormal bone resorption include rheumatoid arthritis, Paget's disease of bone, hypercalcemia, lost of periodontal bone, renal osteodystrophy, osteolytic tumor, bone metastatic tumor and the like. Osteoporosis is particularly appropriate as the disease resulting from the abnormal bone metabolism.

### Examples

### Example 1

### Study on the effect of N-hydroxy-4-{5-[4-(5-isopropyl-2-methyl-1,3-thiazol-4-yl) phenoxy]pentoxy}-benzamidine (Compound 1) and 1α,25-dihydroxyvitamin D₃ on the cell growth of ROS17/2.8 cells (FIG. 1 and Table 2)

ROS17/2.8 cells were inoculated to a 96-well plate at a density of 2 X 10⁴ cells/well. An αMEM medium to which 10% fetal calf serum had been added (referred to as the 10% FCS-αMEM, hereinafter) was used as a medium. After culture of the cells overnight, the medium was removed from each well, and an αMEM medium to which 0.5% fetal calf serum had been added (referred to as the 0.5% FCS-αMEM, hereinafter) was added. After culture of the cells for about six hours, the medium was removed from each well, and a 10% FCS-αMEM comprising DMSO and ethanol (referred to as Solution 1, hereinafter), a Compound 1 solution and ethanol (referred to as Solution 2, hereinafter), a 1α,25-dihydroxyvitamin D₃ solution and DMSO (referred to as Solution 3, hereinafter) or the Compound 1 solution and the 1α,25-dihydroxyvitamin D₃ solution (referred to as Solution 4, hereinafter) was added. The 10% FCS-αMEM comprising Solution 1 was prepared by diluting DMSO and ethanol by 1000 fold with the 10% FCS-αMEM. The 10% FCS-αMEM comprising Solution 2 was prepared by diluting a 10⁻⁵M Compound 1 solution and ethanol by 1000 fold with the 10% FCS-αMEM. The 10% FCS-αMEM comprising Solution 3 was prepared by diluting a 10⁻⁴M 1α,25-dihydroxyvitamin D₃ solution and DMSO by 1000 fold with the 10% FCS-αMEM. The 10% FCS-αMEM comprising Solution 4 was prepared by diluting the 10⁻⁵M Compound 1 solution and the 10⁻⁴M 1α,25-dihydroxyvitamin D₃ solution by 1000 fold with the 10% FCS-αMEM. After culture of the cells for about 24 hours, the effect of Compound 1 and 1α,25-dihydroxyvitamin D₃ on the cell growth of the ROS17/2.8 cells was evaluated using Amersham Cell Proliferation Biotrak ELISA system, version 2 (manufactured by GE healthcare bioscience K.K., Code No. RPN250). Namely, growing cells were made take 5-bromo-2'-deoxyuridine (BrdU) into the cells, and the cells were detected by ELISA using peroxidase-labeled anti-BrdU antibodies. The amount of BrdU taken into the cells was determined by measuring absorbance (450 nm) of the solution by a microplate reader.

The results thereof were shown in FIG. 1. The BrdU uptake amount in each solution addition group was obtained in terms of a percentage to the average value of the BrdU uptake amount in four wells in a Solution 1 addition group. The graph was made using the average value ± standard error of the four wells of each solution addition group. The cell growth of the ROS17/2.8 cells cultured in the presence of the 10% FCS-αMEM comprising Solution 2 was not significantly different, compared with the cell growth of the ROS17/2.8 cells cultured in the presence of the 10% FCS-αMEM comprising Solution 1 (n.s., Dunnett multiple comparison test). In addition, the cell growth of the ROS17/2.8 cells cultured in the presence of the 10% FCS-αMEM comprising Solution 3 was not significantly different, compared with the cell growth of the ROS17/2.8 cells cultured in the presence of the 10% FCS-αMEM comprising Solution 1 (n.s., Dunnett multiple comparison test). On the other hand, the cell growth of the ROS17/2.8 cells cultured in the presence of the 10% FCS-αMEM comprising Solution 4 was significantly increased, compared with the cell growth of the ROS17/2.8 cells cultured in the presence of the 10% FCS-αMEM comprising Solution 1 (**: p<0.01, Dunnett multiple comparison test). Namely, it became clear that stimulation only by the combination of Compound 1 with 1α,25-dihydroxyvitamin D₃ increased the cell growth of the ROS17/2.8 cells.

The results of the study on the synergetic action of Compound 1 and 1α,25-dihydroxyvitamin D₃ on the ROS17/2.8 cell growth by the Bürgi's method (edited by Masajiro TAKAGI, and Hikari OZAWA, "pharmacognosy", p.18-19, Nanzando, 1987) were shown in Table 2. In the Bürgi's method, the value of a standard group is subtracted from the values of each treated group to obtain a relative index. If the value of the combination treatment group is more excellent in comparison of the product of the relative indices in a group treated with a single agent with the relative index of a group treated with the drug combination, it is determined that there is a synergetic action. If they are the same, it is determined that there is an added action. In contrast, if the value of the combination treatment group is poor, it is determined that there is an antagonistic action.

**Table 2**

| | Solution 1 | Solution 2 (Compound 1 alone) | Solution 3 (1α,25-dihydroxyvitamin D₃ alone) | Product of single agent addition group | Solution 4 (Combination group) |
|---|---|---|---|---|---|
| Relative index | 1.000 | 1.011 | 1.220 | 1.233 | 1.596 |

The relative index of the BrudU uptake amount in each solution addition group was calculated, considering the BrdU uptake amount obtained when Solution 1 was added to be 1.000. The relative index (1.596) of a group for which Compound 1 and 1α,25-dihydroxyvitamin D₃ were added in combination was larger than the product (1.233) of relative indices of the single agent addition group, and the synergetic effect resulting from the drug combination was recognized.

### Example 2

### Study on the effect of N-hydroxy-4-5-[4-(2-dipropylamino-5-ethyl-1,3-thiazol-4-yl) phenoxylpentoxy-benzamidine (Compound 11) and 1α,25-dihydroxyvitamin D₃ on the cell growth of ROS17/2.8 cells (FIG. 2 and Table 3)

ROS17/2.8 cells were inoculated to a 96-well plate at a density of 5 X 10³ cells/well. The 10% FCS-αMEM was used as a medium. After culture of the cells overnight, the medium was removed from each well, and the 0.5% FCS-αMEM was added. After culture of the cells for about six hours, the medium was removed from each well, and the 10% FCS-αMEM comprising DMSO and ethanol (referred to as Solution 1, hereinafter), a Compound 11 solution and ethanol (referred to as Solution 5, hereinafter), the 1α,25-dihydroxyvitamin D₃ solution and DMSO (referred to as Solution 3, hereinafter) or the Compound 11 solution and the 1α,25-dihydroxyvitamin D₃ solution (referred to as Solution 6, hereinafter) was added. The 10% FCS-αMEM comprising Solution 1 was prepared by diluting DMSO and ethanol by 1000 fold with the 10% FCS-αMEM. The 10% FCS-αMEM comprising Solution 5 was prepared by diluting a 10⁻⁵M Compound 11 solution and ethanol by 1000 fold with the 10% FCS-αMEM. The 10% FCS-αMEM comprising Solution 3 was prepared by diluting a 10⁻⁴M 1α,25-dihydroxyvitamin D₃ solution and DMSO by 1000 fold with the 10% FCS-αMEM.

The 10% FCS-αMEM comprising Solution 6 was prepared by diluting the 10⁻⁵M Compound 11 solution and the 10⁻⁴M 1α,25-dihydroxyvitamin D₃ solution by 1000 fold with the 10% FCS-αMEM. After culture of the cells for about 24 hours, the effect of Compound 11 and 1α,25-dihydroxyvitamin D₃ on the cell growth of the ROS17/2.8 cells was evaluated using Amersham Cell Proliferation Biotrak ELISA system, version 2 (manufactured by GE healthcare bioscience K.K., Code No. RPN250). Namely, growing cells were made take 5-bromo-2'-deoxyuridine (BrdU) into the cells, and the cells were detected by ELISA using peroxidase-labeled anti-BrdU antibodies. The amount of BrdU taken into the cells was determined by measuring absorbance (450 nm) of the solution by a microplate reader.

The results thereof were shown in FIG. 2. The BrdU uptake amount in each solution addition group was obtained in terms of a percentage to the average value of the BrdU uptake amount in four wells in a Solution 1 addition group. The graph was made using the average value ± standard error of the four wells of each solution addition group. The cell growth of the ROS17/2.8 cells cultured in the presence of the 10% FCS-αMEM comprising Solution 5 was not significantly different, compared with the cell growth of the ROS17/2.8 cells cultured in the presence of the 10% FCS-αMEM comprising Solution 1 (n.s., Dunnett multiple comparison test). In addition, the cell growth of the ROS17/2.8 cells cultured in the presence of the 10% FCS-αMEM comprising Solution 3 was not significantly different, compared with the cell growth of the ROS17/2.8 cells cultured in the presence of the 10% FCS-αMEM comprising Solution 1 (n.s., Dunnett multiple comparison test). On the other hand, the cell growth of the ROS17/2.8 cells cultured in the presence of the 10% FCS-αMEM comprising Solution 6 was not significantly different, compared with the cell growth of the ROS17/2.8 cells cultured in the presence of the 10% FCS-αMEM comprising Solution 1, but a tendency of clear induction of cell growth was recognized (p=0.066, Dunnett multiple comparison test).

The results of the study on the synergetic action of Compound 11 and 1α,25-dihydroxyvitamin D₃ on the ROS17/2.8 cell growth by the Bürgi's method were shown in Table 3. The relative index of the BrdU uptake amount of each solution addition group was calculated, considering the BrdU uptake amount obtained when Solution 1 was added to be 1.000. The relative index (1.444) of the group for which Compound 11 and 1α,25-dihydroxyvitamin D₃ were added in combination was larger than the product (1.223) of the relative indices of each single agent addition group, and a synergetic effect by the drug combination was recognized.

**Table 3**

| | Solution 1 | Solution 5 (Compound 11 alone) | Solution 3 (1α, 25-dihydroxyvitamin Singe D₃ alone) | Product of relative indices of agent addition group | Solution 6 (Combination group) |
|---|---|---|---|---|---|
| Relative index | 1.000 | 1.023 | 1.195 | 1.223 | 1.444 |

### Example 3

### Study on the effect of an N-hydroxy benzamidine derivative (referred to as Test Compound, hereinafter) listed in Table 1, and 1α,25-dihydroxyvitamin D₃ on the cell growth of the ROS17/2.8 cells (Tables 4 and 5)

100 µL of a 10% FCS-αMEM comprising either DMSO and ethanol (referred to as Solution 7, hereinafter), a Test Compound solution and ethanol (referred to as Solution 8, hereinafter), a 1α,25-dihydroxyvitamin D₃ solution and DMSO (referred to as Solution 9, hereinafter) or a Test Compound solution and the 1α,25-dihydroxyvitamin D₃ solution (referred to as Solution 10, hereinafter) was dispensed. The ROS17/2.8 cells were inoculated to a medium at a density of 5 x 10³ cells/100 µL/well. After culture of the cells for about 48 hours, the effect of Test Compound and 1α,25-dihydroxyvitamin D₃ on the cell growth of the ROS17/2.8 cells was evaluated using the MTT method. Namely, after culture of the cells for about 44 hours, an MTT solution (3-[4,5-dimethylthiazol-2-yl]-2,5-diphenyltetrazolium bromide 2 mg/mL) was added in an amount of 25 µL/well, and the cells were further cultured for four hours. After the medium was removed, DMSO was added in an amount of 100 µL/well so as to dissolve the formed formazan. The amount of the formed formazan was determined by measuring the absorbance (at 560 nm) of the solution by a microplate reader.

The 10% FCS-αMEM comprising Solution 7 was prepared by diluting DMSO by 1000 fold with the 10% FCS-αMEM, and diluting ethanol by 10000 fold with the 10% FCS-αMEM. The 10% FCS-αMEM comprising Solution 8 was prepared by diluting a 2 x 10⁻⁵M Test Compound solution by 1000 fold with the 10% FCS-αMEM, and diluting ethanol by 10000 fold with the 10% FCS-αMEM. The 10% FCS-αMEM comprising Solution 9 was prepared by diluting 2 x 10⁻³M 1α,25-dihydroxyvitamin D₃ solution by 10000 fold with the 10% FCS-αMEM, and diluting DMSO by 1000 fold with the 10% FCS-αMEM. The 10% FCS-αMEM comprising Solution 10 was prepared by diluting a 2 x 10⁻⁵M Test Compound solution by 1000 fold with the 10% FCS-αMEM, and diluting 2 x 10⁻³M 1α,25-dihydroxyvitamin D₃ solution by 10000 fold with the 10% FCS-αMEM.

The results thereof were shown in Table 4. The amount of the formed formazan in each solution addition group was obtained in terms of a percentage to the average value of the amounts of formed formazan in four wells in a Solution 7 addition group. All the determined values were shown in terms of the average value ± standard deviation of the four wells of each solution addition group. It was recognized that the cell growth of the ROS17/2.8 cells cultured in the presence of the 10% FCS-αMEM comprising Solution 10 which comprises both the agents was significantly different, compared with the cell growth of the ROS17/2.8 cells cultured in the presence of the 10% FCS-αMEM comprising either Solution 8 or Solution 9 (*; p<0.05, **; p<0.01 vs. a single Test Compound addition group, Student's t test, #; p<0.05, ##; p<0.01 vs. a single 1α,25-dihydroxyvitamin D₃ addition group, Student's t test).

**Table 4**

| Compound No. | Solution 7 (Solvent control) | Solution 8 (Test Compound alone) | Solution 9 (1α,25-dihydroxyvitamin D₃ alone) | Solution 10 (a group of a drug combination) |
|---|---|---|---|---|
| Control | 100±3.0 | | 105±3.1 | |
| 2 | | 103±3.1 | | 117±2.6*,# |
| 3 | | 105±3.1 | | 112±2.7 *,# |
| 4 | | 105±1.9 | | 112±1.6*,# |
| 5 | | 100±3.7 | | 110±2.4*,# |
| 6 | | 103±2.5 | | 113±1.7 **,## |
| 7 | | 109±3.2 | | 120±2.3**,## |
| 8 | | 102±4.9 | | 115±2.3**,## |
| 9 | | 108±2.3 | | 120±2.1 **,## |
| 10 | | 99±2.5 | | 111±1.3 **,# |
| 11 | | 103±2.4 | | 115±4.4**,# |
| 12 | | 105±3.0 | | 115±4.3 *,# |
| 13 | | 103±5.2 | | 113±2.1 *,# |
| 14 | | 103±2.5 | | 113±1.3 *,# |
| 15 | | 103±3.0 | | 115±0.8**,## |
| 16 | | 107±1.3 | | 118±3.7**,# |
| 17 | | 97±1.3 | | 113±1.7**,# |
| 18 | | 106±5.0 | | 115±3.0 *,## |
| 19 | | 108±2.7 | | 119±4.8*,# |
| 20 | | 106±3.1 | | 113±1.0*,## |
| 21 | | 105±2.5 | | 111±1.7 *,# |
| 22 | | 110±4.1 | | 122±2.1**,## |
| 23 | | 107±5.5 | | 123±10.2*,# |
| 24 | | 110±4.4 | | 118±2.9*,## |
| 25 | | 101±3.2 | | 112±1.5 *,# |
| 26 | | 102±2.6 | | 113±2.7 *,# |
| 27 | | 98±1.9 | | 112±0.9 **,## |
| 28 | | 99±3.5 | | 111±2.0 *,# |
| 29 | | 102±5.6 | | 114±2.6 *,## |
| 30 | | 99±5.3 | | 113±1.2 *,## |
| 31 | | 101±2.9 | | 110±0.4 *,# |
| 32 | | 97±3.3 | | 110±2.4 **,# |
| 33 | | 106±1.7 | | 113±1.9 **,## |
| 34 | | 105±3.6 | | 112±3.7 *,# |
| 35 | | 101±2.3 | | 112±3.0*,## |

The results of the study on the synergetic action of Test Compound and 1α,25-dihydroxyvitamin D₃ on the ROS17/2.8 cell growth were shown in Table 5. The relative index of the amount of formed formazan in each solution addition group was calculated, considering the amount of the formed formazan obtained when Solution 7 was added to be 1.00. The relative index of the group for which Test Compound and 1α,25-dihydroxyvitamin D₃ were administered in combination was larger than the product of relative indices of the single agent addition group, and the synergetic effect was recognized in all the combination of Test Compound with 1α,25-dihydroxyvitamin D₃.

**Table 5**

| Compound No. | Relative index of Solution 7 (Solvent control) | Relative index of Solution 8 (Test Compound alone) | Relative index of Solution 9 (1α,25-dihydroxyvitamin D₃ alone | Product of relative indices of single agent addition group | Relative index of Solution 10 (a group of a drug combination) |
|---|---|---|---|---|---|
| Control | 1.00 | | 1.05 | | |
| 2 | | 1.03 | | 1.08 | 1.17 |
| 3 | | 1.05 | | 1.10 | 1.12 |
| 4 | | 1.05 | | 1.10 | 1.12 |
| 5 | | 1.00 | | 1.05 | 1.10 |
| 6 | | 1.03 | | 1.08 | 1.13 |
| 7 | | 1.09 | | 1.14 | 1.20 |
| 8 | | 1.02 | | 1.07 | 1.15 |
| 9 | | 1.08 | | 1.13 | 1.20 |
| 10 | | 0.99 | | 1.04 | 1.11 |
| 11 | | 1.03 | | 1.08 | 1.15 |
| 12 | | 1.05 | | 1.10 | 1.15 |
| 13 | | 1.03 | | 1.08 | 1.13 |
| 14 | | 1.03 | | 1.08 | 1.13 |
| 15 | | 1.03 | | 1.08 | 1.15 |
| 16 | | 1.07 | | 1.12 | 1.18 |
| 17 | | 0.97 | | 1.02 | 1.13 |
| 18 | | 1.06 | | 1.11 | 1.15 |
| 19 | | 1.08 | | 1.13 | 1.19 |
| 20 | | 1.06 | | 1.11 | 1.13 |
| 21 | | 1.05 | | 1.10 | 1.11 |
| 22 | | 1.10 | | 1.16 | 1.22 |
| 23 | | 1.07 | | 1.12 | 1.23 |
| 24 | | 1.10 | | 1.16 | 1.18 |
| 25 | | 1.01 | | 1.06 | 1.12 |
| 26 | | 1.02 | | 1.07 | 1.13 |
| 27 | | 0.98 | | 1.03 | 1.12 |
| 28 | | 0.99 | | 1.04 | 1.11 |
| 29 | | 1.02 | | 1.07 | 1.14 |
| 30 | | 0.99 | | 1.04 | 1.13 |
| 31 | | 1.01 | | 1.06 | 1.10 |
| 32 | | 0.97 | | 1.02 | 1.10 |
| 33 | | 1.06 | | 1.11 | 1.13 |
| 34 | | 1.05 | | 1.10 | 1.12 |
| 35 | | 1.01 | | 1.06 | 1.12 |

New bone constantly replaces existing bone by osteoblast involved in osteogenesis and osteoclast involved in bone destruction to maintain the normal levels of bone quality and mass. It is a known fact that the bone mass is increased when the balance of the amount or function level of osteoblast and osteoclast is such that osteogenesis is relatively increased (Written and edited by Tatsuo SUDA, Hidehiro OZAWA, Hideaki TAKAHASHI, Sakae TANAKA, Hiroaki NAKAMURA, and Satoshi MORI, "Science of Bone", Ishiyaku Shuppan K.K., 2007). In particular, it is presumed that an increase in osteoblast amount and function is directly linked to promotion of osteogenesis. The ROS17/2.8 cells used in Examples of the specification of the present application are osteoblast-like cells derived from osteosarcoma, and thus it can be said that an increase in the cells would promote the osteogenesis action. Consequently, these results suggested that a combination of an N-hydroxybenzamidine derivative represented by Formula (I) or a salt thereof with the activated vitamin D or a prodrug thereof exhibits a strong osteogenesis promoting action, compared with the use of each compound alone.

### Industrial Applicability

A combination of the N-hydroxybenzamidine derivative represented by Formula (I) or a salt thereof with the activated vitamin D or a prodrug thereof of the present invention can be used as a therapeutic agent or a prophylactic agent, or a therapeutic method or a prophylactic method for a disease resulting from an abnormal bone metabolism, especially osteoporosis.

## Claims

1. A therapeutic agent or a prophylactic agent for a disease resulting from an abnormal bone metabolism comprising the following (a) and (b) as active ingredients:
(a) an N-hydroxybenzamidine derivative represented by the following Formula (I) or a salt thereof; and
(b) an activated vitamin D or a prodrug thereof: 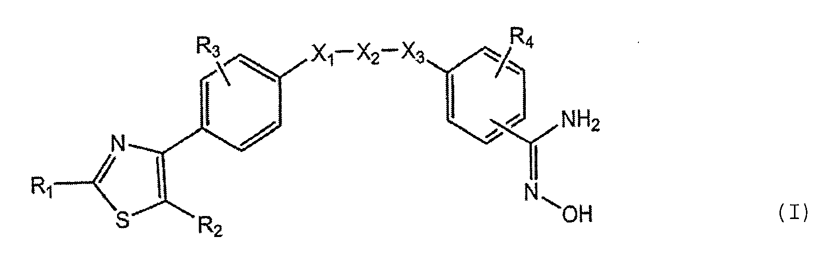 [In the above Formula (I),
R¹ is hydrogen; C1-C6 alkyl; C3-C6 cycloalkyl; C6-C12 aryl, 5- to 12-membered heteroaryl ring containing 1 to 3 heteroatoms selected from N, O and S; or NR⁵R⁶;
R² is hydrogen; C1-C6 alkyl; C2-C6 alkenyl; C3-C6 cycloalkyl; amino; C1-3 dialkylamino; C1-3 alkyl substituted by halogen, C3-C6 cycloalkyl, C6-C12 aryl, 5-to 12-membered heteroaryl ring containing 1 to 3 heteroatoms selected from N, O and S, or C3-6 heterocycloalkyl containing 1 to 3 heteroatoms selected from N, O and S; C1-3 alkyl substituted by NR⁷R⁸; or N-methylcarbamoyl;
R³ and R⁴ independently are hydrogen; halogen; hydroxy; C1-6 alkyl which is unsubstituted or is substituted by a halogen; C3-6 cycloalkylamino; C1-6 alkoxy; C1-6 alkanoyloxy; C2-6 alkenyloxy; phenyl-C1-6 alkoxy; phenoxy; C2-6 alkenoyloxy; phenyl-C1-6 alkanoyloxy; C3-6 cycloalkyloxy substituted by carboxy, esterified carboxy or amidated carboxy; or aminooxy;
R⁵ is hydrogen or C1-C6 alkyl;
R⁶ is hydrogen; C1-C6 alkyl; C1-3 alkyl substituted by hydroxy, C6-C12 aryl, 5- to 12-membered heteroaryl ring containing 1 to 3 heteroatoms selected from N, O and S, or C3-6 heterocycloalkyl containing 1 to 3 heteroatoms selected from N, O and S;
R⁷ is hydrogen; C1-C6 alkyl; or C3-C6 cycloalkyl;
R⁸ is hydrogen; C1-C4 alkyl; C1-3 alkyl substituted by C1-3 alkoxy; or 4-pyridinoyl;
X¹ is -O-; -S-; -NH-; or -N(C1-6 alkyl)-;
x² is C3-7 alkylene; C3-7 alkylenecarbonyl; or C1-3 alkylene-phenylene-C1-3 alkylene;
X³ is -O -S-; -NH-; or -N(C1-6 alkyl)-;].

2. The therapeutic agent or the prophylactic agent for a disease resulting from the abnormal bone metabolism as described in claim 1, wherein
1) when R¹ is C1-C6 alkyl,
R² is C1-C6 alkyl; C2-C6 alkenyl; C3-C6 cycloalkyl; amino; C1-3 dialkylamino; C1-3 alkyl substituted by halogen, C3-C6 cycloalkyl, C6-C12 aryl, 5- to 12-membered heteroaryl ring containing 1 to 3 heteroatoms selected from N, O and S, or C3-6 heterocycloalkyl containing 1 to 3 heteroatoms selected from N, O and S; or C1-3 alkyl substituted by NR⁷R⁸;
2) when R¹ is C3-C6 cycloalkyl,
R² is C1-C6 alkyl; or C1-3 alkyl substituted by NR⁷R⁸;
3) when R¹ is C6-C12 aryl or 5- to 12-membered heteroaryl ring containing 1 to 3 heteroatoms selected from N, O and S,
R² is C1-C3 alkyl substituted by halogen, C3-C6 cycloalkyl, C6-C12 aryl, 5- to 12-membered heteroaryl ring containing 1 to 3 heteroatoms selected from N, 0 and S, or C3-6 heterocycloalkyl containing 1 to 3 heteroatoms selected from N, O and S;
4) when R¹ is NR⁵R⁶,
R² is C1-C6 alkyl; C3-6 cycloalkyl; C1-C3 alkyl substituted by halogen, C3-C6 cycloalkyl, C6-C12 aryl, 5-to 12-membered heteroaryl ring containing 1 to 3 heteroatoms selected from N, O and S, or C3-6 heterocycloalkyl containing 1 to 3 heteroatoms selected from N, O and S; or N-methylcarbamoyl.

3. The therapeutic agent or the prophylactic agent for a disease resulting from the abnormal bone metabolism as described in claim 1 or 2, wherein
1) when R⁵ is hydrogen,
R⁶ is hydrogen; or C1-C6 alkyl;
2) when R⁵ is C1-C6 alkyl,
R⁶ is C1-C6 alkyl; C1-3 alkyl substituted by a group selected from hydroxy, C6-C12 aryl, 5- to 12-membered heteroaryl ring containing 1 to 3 heteroatoms selected from N, O and S, and C3-6 heterocycloalkyl containing 1 to 3 heteroatoms selected from N, O and S.

4. The therapeutic agent or the prophylactic agent for a disease resulting from the abnormal bone metabolism as described in any one of claims 1 to 3, wherein
1) when R⁷ is hydrogen,
R⁸ is C1-C4 alkyl; or C1-3 alkyl substituted by C1-3 alkoxy or C3-6 heterocycloakyl;
2) when R⁷ is C1-C6 alkyl,
R⁸ is C1-C4 alkyl;
3) when R⁷ is C3-C6 cycloalkyl,
R⁸ is 4-pyridinoyl.

5. The therapeutic agent or the prophylactic agent for a disease resulting from the abnormal bone metabolism as described in any one of claims 1 to 4, wherein R¹ is hydrogen; methyl; ethyl; propyl; isopropyl; cyclohexyl; phenyl; 3-pyridinyl; or NR⁵R⁶.

6. The therapeutic agent or the prophylactic agent for a disease resulting from the abnormal bone metabolism as described in any one of claims 1 to 5, wherein
R² is hydrogen; methyl; ethyl; propyl; isopropyl; butyl; vinyl; cyclopentyl; amino; dimethylamino; methyl or ethyl substituted by chlorine, pentyl, phenyl, 1-imidazolyl, 4-thiomorpholinyl, 4-morpholinyl, or NR⁷R⁸; or methyl or ethyl substituted by N-methylcarbamoyl.

7. The therapeutic agent or the prophylactic agent for a disease resulting from the abnormal bone metabolism as described in any one of claim 1 to 6, wherein R³ is hydrogen.

8. The therapeutic agent or the prophylactic agent for a disease resulting from the abnormal bone metabolism as described in any one of claims 1 to 7, wherein R⁴ is hydrogen; or fluorine.

9. The therapeutic agent or the prophylactic agent for a disease resulting from the abnormal bone metabolism as described in any one of claims 1 to 8, wherein R⁵ is hydrogen; methyl; ethyl; propyl; or isopropyl.

10. The therapeutic agent or the prophylactic agent for a disease resulting from the abnormal bone metabolism as described in any one of claims 1 to 9, wherein R⁶ is hydrogen; methyl; ethyl; propyl; isopropyl; or methyl or ethyl substituted by hydroxy, phenyl, 3-pyridinyl or 4-morpholinyl.

11. The therapeutic agent or the prophylactic agent for a disease resulting from the abnormal bone metabolism as described in any one of claims 1 to 10, wherein R⁷ is hydrogen; methyl; ethyl or cyclopropyl.

12. The therapeutic agent or the prophylactic agent for a disease resulting from the abnormal bone metabolism as described in any one of claims 1 to 11, wherein R⁸ is hydrogen; methyl; ethyl; isopropyl; 3-isopropyloxypropyl; or 4-pyridinoyl.

13. The therapeutic agent or the prophylactic agent for a disease resulting from the abnormal bone metabolism as described in any one of claims 1 to 12, wherein
X¹ is O;
X² is butylene; pentylene; 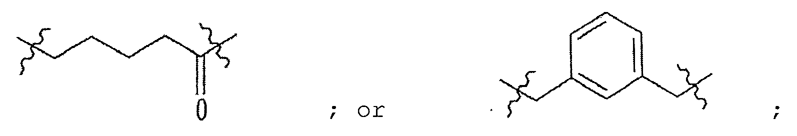
X³ is -O-; -NH-; or -N(CH3)-.

14. The therapeutic agent or the prophylactic agent for a disease resulting from the abnormal bone metabolism as described in any one of claims 1 to 13, wherein
X¹-X²-X³ is 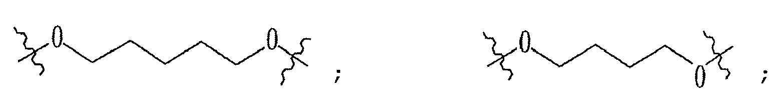 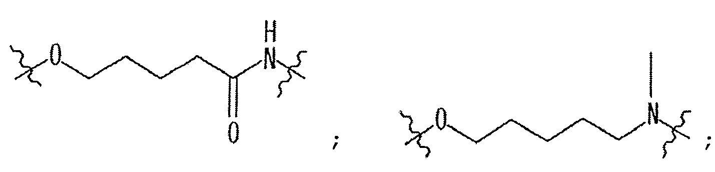 or

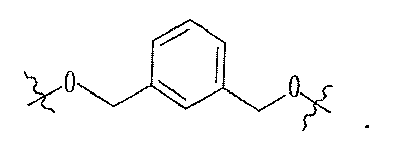

15. The therapeutic agent or the prophylactic agent for a disease resulting from the abnormal bone metabolism as described in claim 1, wherein the N-hydroxybenzamidine derivative represented by Formula (I) is any one of the following compounds:
1) N-hydroxy-4-{5-[4-(5-isopropyl-2-methyl-1,3-thiazol-4-yl)phenoxy]pentoxy}benzamidine
2) N-hydroxy-4-{5-[4-(5-benzyl-2-(pyridin-3-yl)-1,3-thiazol-4-yl]phenoxy]pentoxy}benzamidine
3) N-hydroxy-4-{5-[4-(5-(2-chloroethyl)-2-methyl-1,3-thiazol-4-yl)phenoxy]pentoxy}benzamidine
4) N-hydroxy-4-{5-[4-(5-cyclopentylmethyl-2-ethyl-1,3-thiazol-4-yl)phenoxy]pentoxy}benzamidine
5) N-hydroxy-4-{5-[4-(5-vinyl-2-methyl-1,3-thiazol-4-yl)phenoxy]pentoxy}benzamidine
6) N-hydroxy-4-{5-[4-(5-cyclopentylmethyl-2-methylamino-1,3-thiazol-4-yl)phenoxy]pentoxy}benzamidine
7) N-hydroxy-4-{5-[4-(2-(2-hydroxyethyl)methylamino-5-methyl-1,3-thiazol-4-yl)phenoxy]pentoxy}benzamidine
8) N-hydroxy-4-{5-[4-(2-benzylmethylamino-5-methyl-1,3-thiazol-4-yl)phenoxy]pentoxy}benzamidine
9) N-hydroxy-4-{5-[4-(5-methyl-2-(methyl(pyridin-3-ylmethyl)amino)-1,3-thiazol-4-yl)phenoxy]pentoxy}benzamidine
10) N-hydroxy-4-{5-[4-(2-benzylmethylamino-5-ethyl-1,3-thiazol-4-yl)phenoxy]pentoxy}benzamidine
11) N-hydroxy-4-{5-[4-(2-dipropylamino-5-ethyl-1,3-thiazol-4-yl)phenoxy]pentoxy}benzamidine
12) N-hydroxy-4-{5-[4-(5-isopropyl-2-(methyl(pyridin-3-yl-methyl)amino)-1,3-thiazol-4-yl)phenoxy]pentoxy}benzamidine
13) N-hydroxy-4-{5-[4-(5-butyl-2-(methyl(2-(4-morpholino)ethyl)amino)-1,3-thiazol-4-yl)phenoxy]pentoxy}benzamidine
14) N-hydroxy-4-{5-[4-(5-cyclopentylmethyl-2-dipropylamino-1,3-thiazol-4-yl)phenoxy]pentoxy}benzamidine
15) N-hydroxy-4-{5-[4-(5-cyclopentyl-2-(methyl(2-(4-morpholino)ethyl)amino)-1,3-thiazol-4-yl)phenoxy]pentoxy}benzamidine
16) N-hydroxy-4-{5-[4-(5-isopropyl-2-dipropylamino-1,3-thiazol-4-yl)phenoxy]pentoxy}benzamidine
17) N-hydroxy-4-{5-[4-(5-isopropyl-2-dimethylamino-1,3-thiazol-4-yl)phenoxy]pentoxy}benzamidine
18) N-hydroxy-4-{5-[4-(5-isopropyl-2-methyl-1,3-thiazol-4-yl)phenoxy]pentanoylamino}benzamidine
19) N-hydroxy-4-{5-[4-(5-isopropyl-2-methyl-1,3-thiazol-4-yl)phenoxy]pentylmethylamino}benzamidine
20) N-hydroxy-2-fluoro-4-{5-[4-(5-isopropyl-2-methyl-1,3-thiazol-4-yl)phenoxy]pentoxy}benzamidine
21) N-hydroxy-4-{4-[4-(2-cyclohexyl-5-ethyl-1,3-thiazol-4-yl)phenoxy]butoxy}benzamidine
22) N-hydroxy-4-{3-[4-(5-isopropyl-2-methyl-1,3-thiazol-4-yl)phenoxymethyl]benzyloxy}benzamidine
23) N-hydroxy-4-{5-[4-(5-(2-(imidazol-1-yl)ethyl)-2-methyl-1,3-thiazol-4-yl)phenoxy]pentoxy}benzamidine
24) N-hydroxy-4-{5-[4-(5-(2-(isopropylamino)ethyl)-2-methyl-1,3-thiazol-4-yl)phenoxy]pentoxy}benzamidine
25) N-hydroxy-4-{5-[4-(5-(2-(3-(isopropoxy)propylamino)ethyl)-2-methyl-1,3-thiazol-4-yl)phenoxy]pentoxy}benzamidine
26) N-hydroxy-4-{5-[4-(5-(2-diethylaminoethyl)-2-methyl-1,3-thiazol-4-yl)phenoxy]pentoxy}benzamidine
27) N-hydroxy-4-{5-[4-(2-isopropyl-5-(2-(thiomorpholin-4-yl)ethyl)-1,3-thiazol-4-yl)phenoxy]pentoxy}benzamidine
28) N-hydroxy-4-{5-[4-(2-cyclohexyl-5-(2-(dimethylamino)ethyl)-1,3-thiazol-4-yl)phenoxy]pentoxy}benzamidine
29) N-hydroxy-4-{5-[4-(2-dimethylamino-5-(2-(morpholin-4-yl)ethyl)-1,3-thiazol-4-yl)phenoxy]pentoxy}benzamidine
30) N-hydroxy-4-{5-[4-(5-(2-(cyclopropyl(pyridin-4-carbonyl)amino)ethyl)-2-isopropyl-1,3-thiazol-4-yl)phenoxy]pentoxy}benzamidine
31) N-hydroxy-4-{5-[4-(2-amino-5-(N-methylcarbamoyl)-1,3-thiazol-4-yl)phenoxy]pentoxy}benzamidine
32) N-hydroxy-4-{5-[4-(5-(imidazol-1-ylmethyl)-2-methyl-1,3-thiazol-4-yl)phenoxy]pentoxy}benzamidine
33) N-hydroxy-4-{5-[4-(2-ethylmethylamino-5-(morpholin-4-ylmethyl)-1,3-thiazol-4-yl)phenoxy]pentoxy}benzamidine
34) N-hydroxy-4-{5-{4-(2-(methyl(2-morpholin-4-yl)ethyl)amino)-5-(thiomorpholin-4-yl)-1,3-thiazol-4-yl)phenoxy]pentoxy}benzamidine
35) N-hydroxy-4-{5-[4-(5-dimethylamino-2-methyl-1,3-thiazol-4-yl)phenoxy]pentoxy}benzamidine.

16. The therapeutic agent or the prophylactic agent for a disease resulting from the abnormal bone metabolism as described in claim 1, wherein the N-hydroxybenzamidine derivative represented by Formula (I) is N-hydroxy-4-{5-[4-(5-isopropyl-2-methyl-1,3-thiazol-4-yl)phenoxy]pentoxy}benzamidine.

17. The therapeutic agent or the prophylactic agent for a disease resulting from the abnormal bone metabolism as described in any one of claims 1 to 16, wherein the disease resulting from the abnormal bone metabolism is a disease resulting from abnormal bone resorption.

18. The therapeutic agent or the prophylactic agent for a disease resulting from the abnormal bone metabolism as described in any one of claims 1 to 16, wherein the disease resulting from the abnormal bone metabolism is disease resulting from abnormal bone formation.

19. The therapeutic agent or the prophylactic agent for a disease resulting from the abnormal bone metabolism as described in any one of claims 1 to 16, wherein the disease resulting from the abnormal bone metabolism is osteoporosis.

20. The therapeutic agent or the prophylactic agent for a disease resulting from the abnormal bone metabolism as described in any one of claims 1 to 19, wherein (b) is 1α,25-dihydroxyvitamin D₃, 1α,24R-dihydroxyvitamin D₃, or 1α-hydroxyvitamin D₃.

21. The therapeutic agent or the prophylactic agent for a disease resulting from the abnormal bone metabolism as described in any one of claims 1 to 19, wherein (b) is 1α,25-dihydroxyvitamin D₃ or 1α-hydroxyvitamin D₃.

22. The therapeutic agent or the prophylactic agent for a disease resulting from the abnormal bone metabolism as described in any one of claims 1 to 21, wherein (a) and (b) form a drug combination.

23. The therapeutic agent or the prophylactic agent for a disease resulting from the abnormal bone metabolism as described in any one of claims 1 to 21, wherein (a) and (b) each is an independent single agent.

24. The therapeutic agent or the prophylactic agent for a disease resulting from the abnormal bone metabolism as described in any one of claims 1 to 21, which is a kit comprising (a) and (b).
